# EUROPEAN PATENT APPLICATION

(11) **EP 4 052 654 A1**
(43) Date of publication of application: **07.09.2022**
(21) Application number: 21211459.9
(22) Date of filing: 02.01.2018
(51) Int. Cl.: A61B 10/00, G01N 33/493, G01N 33/70, G01N 27/414, G01N 33/487

(54) **APPARATUS FOR MEASURING URINE CONTENT**

(30) Priority: 30.12.2016 US 201615395337
(62) Divisional of application: 18700260.5
(71) Applicant: Withings, 92130 Issy-les-Moulineaux (FR)
(72) Inventor: BARAKAT, Christelle, 75005 Paris (FR); BUARD, Nadine, 92190 Meudon (FR); CARREEL, Eric, 92190 Meudon (FR); HUTCHINGS, Cedric, 92130 Issy les Moulineaux (FR)
(74) Representative: Descazeaux, Charles

(57) **Abstract**

A urine analyzer device (1), configured to be installed in a toilet bowl (10), to be used several times, the device comprising a holder member (9), to attach the device to the rim of the toilet bowl, an electronic unit (4), enclosed in a housing, a urine reception area (2), with at least one electro-chemical sensor (3), configured to measure a quantity of at least one substance contained in urine, such as physiological compound or chemical component, a wireless coupler (5) to send resulting data to a remote computing device (50),
wherein the electro-chemical sensor (3) comprises at least a ion selective Field Effect Transistor (ISFET) (8), configured to sense levels of one or more physiological ions,
thereby providing a urine analyzer device, readily available at home, simply installed in a toilet bowl, and that can be used several times subsequently.

## Description

### FIELD OF THE INVENTION

The present invention relates to a urine analyser device, which can be used at home; the invention targets especially an autonomous urine analyser device which can be installed in the bowl of the toilet.

### BACKGROUND OF THE DISCLOSURE

As known, urine is an important source of information that reflects the health condition of an individual. Several biological parameters are reflected in urine. Absolute levels of several biological parameters and also evolution over time of several biological parameters are interesting to be obtained from samples of urine. It is a goal to monitor various health parameters, otherwise called 'bio-markers', that can be deduced from periodic urine analysis at home in a non-medical environment. This is generally helpful to help enhance personal wellness and help reduce some risk factors.

Various minor disorders can be detected early by analysis of evolutions and trends of personal biological parameters.

Additionally, dysfunctions such as pancreatic disorders (typically, diabetes), hypohepatia, and kidney disorders, can be detected advantageously in a non-invasive manner by performing quantitative analysis of certain urine constituents, such as glucose, protein, urobilinogen, occult blood and other substances.

Accordingly, there have been proposed in the art toilets having a urine analysis function which are capable of performing sampling and analysis of urine so as to assist the individuals in rendering their health check by making use of toilets provided in at home, without excluding offices and other facilities.

It is known in the art, as taught by US5,720,054 [TOTO], to install a urine analyser device in the bowl of the toilet. However, the known devices are very complicated, requires many connexions, and are expensive and furthermore it does not handle multiple users.

Therefore, there is a need to bring new solutions to provide urine analyser devices to be used at home in the standard toilet.

### SUMMARY OF THE DISCLOSURE

According to a first aspect of the present disclosure, it is proposed:
A urine analyzer device configured to be installed in a toilet bowl, the device comprising:
- a holder member, to attach the device to the toilet bowl,
- an electronic unit, enclosed in a housing,
- a urine reception area, with at least one electro-chemical sensor, configured to measure a quantity of at least one substance contained in urine, such as a physiological compound or a chemical component,
- a coupler configured to send resulting measured data from the at least one electro-chemical sensor to a remote computing device,
   wherein the electro-chemical sensor comprises at least one of:
   an ion selective Field Effect Transistor (ISFET) configured to sense levels of one or more physiological ions;
   an enzyme selective Field Effect Transistor (enFET) configured to sense levels of one or more physiological compounds or chemical components; and
   a 3-electrode sensing unit configured to sense levels of one or more physiological compounds or chemical components.

Thereby there is provided a urine analyzer device, readily available at home, installed in a toilet bowl, and that can be used several times subsequently.

Thanks to the disclosed features, an individual can follow some health indicators like pH of urine, or the concentration of one or more physiological ions in urine.

The measure of the concentration of one above-mentioned substance (like Na+, K+, Ca2+, Mg2+, Cl-..) is advantageously made ***in situ,*** within the urine analyzer device; no additional device nearby the toilet is required.

Thanks to the coupling with a remote internet-enabled device, the user can share some of the collected data with a physician, hospital practitioner, etc...

The remote computing device can be a smartphone, but it should be understood that, instead of a smartphone, any more generally speaking portable electronic device can also be used (such as a tablet, a phablet, a PDA, a laptop computer, etc).

In various embodiments of the invention, one may possibly have recourse in addition to one and/or other of the following features/arrangements.

According to one option, the ion selective Field Effect Transistor may comprise a reference electrode coupled to a gate port (G) of the transistor, the reference electrode containing a gel electrolyte, the ion selective Field Effect Transistor configured so that when a urine sample is present, the urine sample is interposed between the reference electrode and the gate port (G) of the transistor. This configuration named *"wet configuration"* is simple and reliable.

According to one option, the ion selective Field Effect Transistor may comprise a reference electrode coupled to a gate port (G) of the transistor, the reference electrode containing a powder or a solid compound, the ion selective Field Effect Transistor (8) configured so that when a urine sample is present, the urine sample is interposed between the reference electrode and the gate port (G) of the transistor. This configuration named *"dry configuration"* is easy to prepare and store before use.

According to one option, the ion selective Field Effect Transistor is selective to hydrogen ions, whereby the ion selective Field Effect Transistor comprises an ion sensitive membrane for measuring the pH of a urine sample, so that in the presence of a urine sample, the ion sensitive membrane is interposed between the urine sample and the gate port (G) of the transistor; we note here that in order to maintain the pH of blood in a narrow range [7,35 - 7,45], pH in urine varies in the range [4,5 - 8].

The device may further comprise at least two additional ion selective Field Effect Transistors:
- one for measuring the concentration of Ca2+ ions to determine Calcium level in urine,
- one for measuring the concentration of K+ ions to determine the Potassium level in urine,
   each of additional ion selective Field Effect Transistors comprising a respective ion selective membrane interposed between a urine sample when present and the gate port (G) of the transistor.

Such a triple-sensor configuration turns to be a cost effective solution to follow three of the most important physiological ions.

The device may further comprise at least two ion selective Field Effect Transistors, wherein:
- a first ion selective Field Effect Transistor is configured to measure a concentration of Na+ ions to determine Sodium level in urine,
- a second ion selective Field Effect Transistor is configured to measure a concentration of K+ ions to determine the Potassium level in urine, and
   wherein the first and second ion selective Field Effect Transistors comprise an ion selective membrane for measuring Na+ and K+ respectively, so that in the presence of a urine sample, the ion selective membrane is interposed between the urine sample and the gate port (G) of the respective transistor.

In some, but not necessarily all, example embodiments, the electronic unit may be configured to:
for a urine sample, calculate a ratio of the concentration of Na+ ions to the concentration of K+ ions (Na+ : K+); and
based on the calculated ratio, generate a user feedback for output by one or more of the urine analyser device and a remote user device.

In some, but not necessarily all, example embodiments, the electro-chemical sensor may be configured to be hydrated at least by water from the flush of the toilet.

According to one option, the device may further comprise a calibration solution reservoir arranged above the electro-chemical sensor to periodically provide a drop of calibration solution on the ion selective Field Effect Transistor, preferably one drop every period, said period being comprised between 15 and 45 days. Periodic recalibration enables to compensate for the drift.

The device may comprise two or more electro-chemical sensors, wherein at least two of the electro-chemical sensors can advantageously be connected to a same reference electrode. This further optimizes the cost of the solution.

According to one option, the reception area further comprises as electro chemical sensor at least an (biological) enzyme selective Field Effect Transistor (enFET). One or more membranes each selective to a particular compound enables to sense levels of some products like albumin, glucose, blood cells, nitrites, etc...

The enzyme selective Field Effect Transistor may comprise a reference electrode; this reference electrode may be preferably made from Ag/AgCl. This is easy to prepare and store before use.

The electro-chemical sensor may further comprise a 3-electrode sensing unit, provided with a reference electrode, a working electrode and a counter electrode, all arranged in a screen printed electrode configuration. Mechatronic integration can thus be optimized.

The urine reception area may comprise a porous structure that can be wetted by a flow of urine excreted by a user, and rinsed subsequently by the flush of the toilet. The measure can be performed nearly *on-the-fly;* since a small quantity (but sufficient) of urine is retained by the porous structure. The rinsing can be accompanied by a calibration/recalibration operation.

The urine reception area can form a cup, the device further comprising a controlled discharge valve. -> buffer volume to ease optical sensing.

There may be provided a user feedback formed as a visual, audio or haptic feedback, or any combination thereof.

This provides a Led light signal or a Beep to acknowledge: exemplified messages are "enough urine" / "end of measurement".

The electronic unit 4 may be at least partly housed in the holder member 9. This allows a good mechatronics integration. The device is small, easy to install; The device can be easily removed when cleaning the toilet bowl;

The urine reception area can be movable with respect to the holder member 9 such that the urine reception area can be located closer to the center of the bowl upon actuation from the user. This can be a mechanical linkage or a degree of freedom enabling to displace the urine reception area. Automatic return to a side position is preferred, e.g. when enough urine is received.

The electro chemical sensor may be configured to measure a concentration of one or more of the following substances:
H+, Na+, K+, Ca2+, Mg2+, Cl- , H₂O₂, Glucose, Nitrites, Proteins, Oestrogenes, Luteinine Hormone, Beta-HCG Hormone (pregnancy marker), Creatinine.

Thereby, many health indicators can be followed. Particularly, albumin/protein levels present in urine can be measured and followed over time. Particularly, pregnancy testing can be provided with this device.

The device may comprise at least one enzyme selective Field Effect Transistor (enFET) wherein:
at least one of the enzyme selective Field Effect Transistor is configured to measure a concentration level of Creatinine in a urine sample.

The coupler may be further configured to send the measured Creatinine concentration level to enable the remote computing device to normalize the measured data from the at least one electro-chemical sensor for the same respective urine sample.

The urine analyser device may further comprise an optical sensor 6, configured to measure color and opacity of the urine; Thereby urine density and color index can be inferred as personal biological index(es) to be followed.

The optical sensor may be configured to measure one or more optical properties of a urine sample comprising: the colour of the urine, a level of absorbance of light by the urine, a level of transmittance of light by the urine, and the opacity of the urine; wherein the electronic unit is configured to analyse the measured one or more optical properties of the urine sample and, based on the analysis, associate the urine sample with one of a plurality of predefined concertation levels of Creatinine, thereby generating an estimated Creatinine concertation level for the urine sample.

The coupler may be further configured to send the estimated Creatinine concentration level for the urine sample to enable the remote computing device to normalize the measured data from the at least one electro-chemical sensor for the same respective urine sample.

The urine reception area is detachably mounted to the holder member. (for deep cleaning and/or exchange after N uses)

The device may further comprise a microphone and/or a basic fingerprint sensor arranged on the portion of the device located outside the toilet bowl. For User recognition among several users.

According to a second aspect of the present disclosure, it is proposed a system comprising a urine analyzer device as disclosed above and a toilet seat comprising weight sensing elements and impedance measurement elements, configured to recognise a particular user pattern among a plurality of user patterns, and to allocate results of urine analysis to the recognised particular user. This configuration is convenient for selective management of multiple users.

According to a third aspect of the present disclosure, there is provided a method comprising:
causing at least one electro-chemical sensor to measure a quantity of at least one substance contained in urine, such as a physiological compound or a chemical component;
causing a coupler to send resulting measured data from the at least one electro-chemical sensor to a remote computing device;
wherein the wherein the electro-chemical sensor comprises at least one of:
   an ion selective Field Effect Transistor (ISFET) for sensing levels of one or more physiological ions;
   an enzyme selective Field Effect Transistor (enFET) for sensing levels of one or more physiological compounds or chemical components; and
   a 3-electrode sensing unit for sensing levels of one or more physiological compounds or chemical components.

According to a fourth aspect of the present disclosure, there is provided a computer readable medium comprising computer program code stored thereon, the computer readable medium and computer program code being configured to, when run on at least one processor, perform:
causing at least one electro-chemical sensor to measure a quantity of at least one substance contained in urine, such as a physiological compound or a chemical component;
causing a coupler to send resulting measured data from the at least one electro-chemical sensor to a remote computing device;
wherein the wherein the electro-chemical sensor comprises at least one of:
   an ion selective Field Effect Transistor (ISFET) for sensing levels of one or more physiological ions;
   an enzyme selective Field Effect Transistor (enFET) for sensing levels of one or more physiological compounds or chemical components; and
   a 3-electrode sensing unit for sensing levels of one or more physiological compounds or chemical components.

According to a fifth aspect of the present disclosure, there is provided a urine analyser device comprising:
at least one processor; and
at least one memory including computer program code,
the at least one memory and the computer program code configured to, with the at least one processor, cause the urine analyser device at least to perform:
causing at least one electro-chemical sensor to measure a quantity of at least one substance contained in urine, such as a physiological compound or a chemical component; causing a coupler to send resulting measured data from the at least one electro-chemical sensor to a remote computing device;
wherein the wherein the electro-chemical sensor comprises at least one of:
   an ion selective Field Effect Transistor (ISFET) for sensing levels of one or more physiological ions;
   an enzyme selective Field Effect Transistor (enFET) for sensing levels of one or more physiological compounds or chemical components; and
   a 3-electrode sensing unit for sensing levels of one or more physiological compounds or chemical components.

According to various, but not necessarily all, embodiments of the present disclosure there is provided examples as claimed in the appended claims.

### BRIEF DESCRIPTION OF THE DRAWINGS

Other features and advantages of the present disclosure appear from the following detailed description of one of its embodiments, given by way of non-limiting example, and with reference to the accompanying drawings, in which:
- Figure 1 shows a schematic sectional view of a toilet equipped with a urine analyzer device according to the present disclosure, with two possible embodiments,
- Figure 2 shows a schematic top view of a toilet of Figure 1,
- Figure 3 shows a functional and structural block diagram
- Figure 4 shows a schematic layout and one example of configuration of the device
- Figures 4A and 4B show enlarged views of variants of the reception area with several selective sensing devices,
- Figure 5 shows an overall system view.
- Figures 6A and 6B show a schematic layout of urine analyzer devices with collection position and rest position,
- Figure 7 illustrates diagrammatically a ISFET, i.e. a ion selective Field Effect Transistor,
- Figure 8 illustrates diagrammatically an enFET, i.e. a enzyme selective Field Effect Transistor
- Figure 9 illustrates diagrammatically a triple FET configuration, with a common shared reference electrode.
- Figures 10A and A0B illustrate diagrammatically a 3-electrode configuration, respectively in elevation view and top view.
- Figure 11A illustrates an example of a controller.
- Figure 11B illustrates an example of a delivery mechanism for a computer program.

### DETAILLED DESCRIPTION OF THE DISCLOSURE

In the figures, the same references denote identical or similar elements.

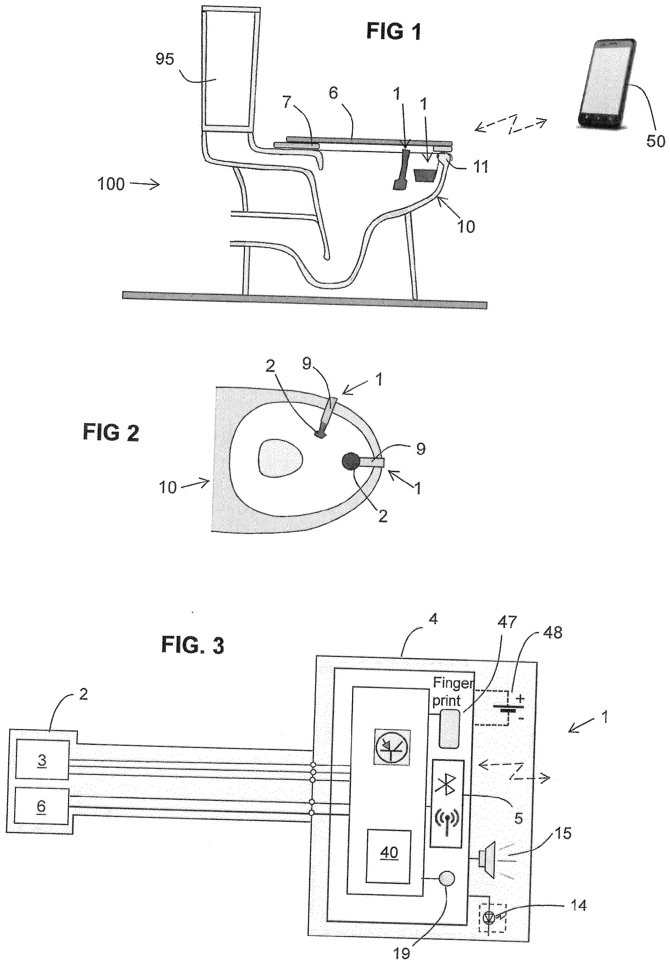

Figures 1 and 2 show a toilet **100** whose overall structure is known. Conventionally, the toilet comprises a water reservoir **95,** a **bowl 10,** a toilet **seat 7** and a toilet **cover 6.**

Further, there is provided a urine analyzer device **1.** For the sake of explanation, there are shown two different devices in the illustration given in figures 1 and 2, but of course the toilet can be equipped with only one urine analyzer device.

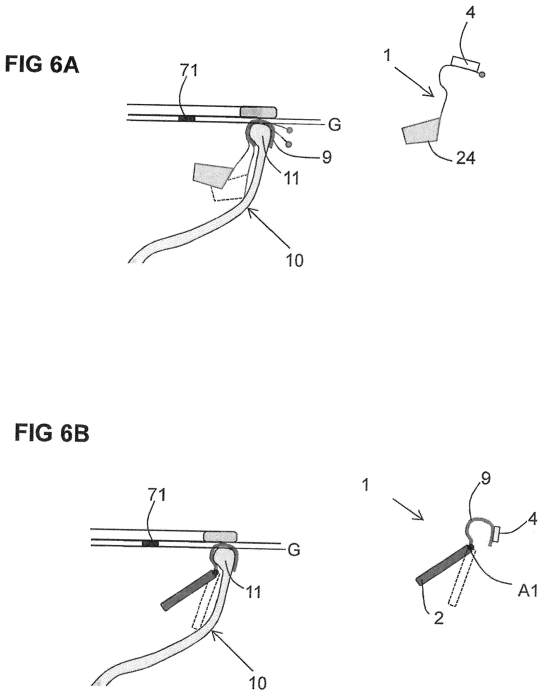

This urine analyzer device may be removably attached to the rim **11** of the bowl. To this end, the urine analyzer device comprises a holder member **9,** to attach the device to the rim of the toilet bowl. The holder member **9** exhibits a width comprised between 3cm and 10cm, and a small thickness to lodge between the upper border of the bowl rim **11** and the lower border of the seat **7,** in other words the thickness is slightly smaller than the available gap **G** defined by the seat spacer pads **71.** (see FIGs 6A & 6B).

The urine analyzer device comprises a **urine reception area** 2, located inside the bowl.

There may be provided a possibility of movement of the reception area 2 with regard to the bowl body, toward the center of the bowl, which will be detailed later. The urine reception area 2 is arranged to be located, when it is at rest position in the peripheral flow of flushing water, for rinsing purpose and possibly recalibration purpose.

The urine analyzer device comprises an **electronic unit** 4, enclosed in a housing. The housing is preferably watertight. The urine analyzer device comprises a **battery 48,** a coupler **5,** for example a wireless radio frequency (RF) coupler such as Bluetooth wireless coupler, an indicator Led **14** and possibly an small loudspeaker or beeper/buzzer **15.**

Additionally, there may be provided a microphone **19,** a fingerprint pad **47** whose purpose will be given later.

According to one embodiment, there is provided a housing in a monolithic fashion, which includes, besides the electronic unit **4,** also the battery and an extension member bearing the urine reception area **2.**

According to another embodiment, the holder member **9** can be distinct from the housing including the electronic unit, the housing being attached in a fixed manner or in articulated manner to the holder member **9.** The electronic unit **4** can be arranged with the holder member **9** in a single housing.

The device according the present disclosure is rather small, and its weight is less than 200 g, preferably less than 150 g.

The urine reception area 2 is permeable to water and/or urine.

The urine reception area 2 comprises at least a **electro-chemical sensor** 3. The electro-chemical electrode sensor 3 is configured to measure a concentratoin of at least one chemical substance, such as a physiological compound or other chemical component including ions, contained in urine.

The term *"physiological compound"* should be understood as any chemical species naturally contained in the human or animal body, from the simplest ions to proteins and amino acids.

The term *"chemical component"* should be understood as some chemical species that are not naturally contained in the human or animal body, like drugs, lead (Pb), mercury (Hg) and so on.

Among the substances which are targeted are the following:
- hydrogen ions (reflecting pH index)
- Sodium, Potassium, Calcium, Magnesium, Chloride,
- H₂O₂ (Hydrogen peroxide)
- Glucose / Proteins / Oestrogenes / luteinine Hormone / Beta-HCG Hormone (pregnancy marker) / Creatinin / PSA Antigen (prostate disorder marker).

As some examples among others, here below are the range and sensitivity of electro-chemical sensor with regard to particular substances:

| | | |
|---|---|---|
| luteinine Hormone | from 0,2 mUl/ml to 80 mUl/ml | sensitivity: 0,2 mUl/ml |
| Creatinin | from 5 mg/ml to 20 mg/ml | sensitivity: 1 mg/ml |
| Albumin | from 75 µg/ml to 750 µg/ml | sensitivity: 30 µg /ml |

Among the physiological ions which are targeted are the following: H+, Na+, K+, Mg2+, Ca2+, Cl-.

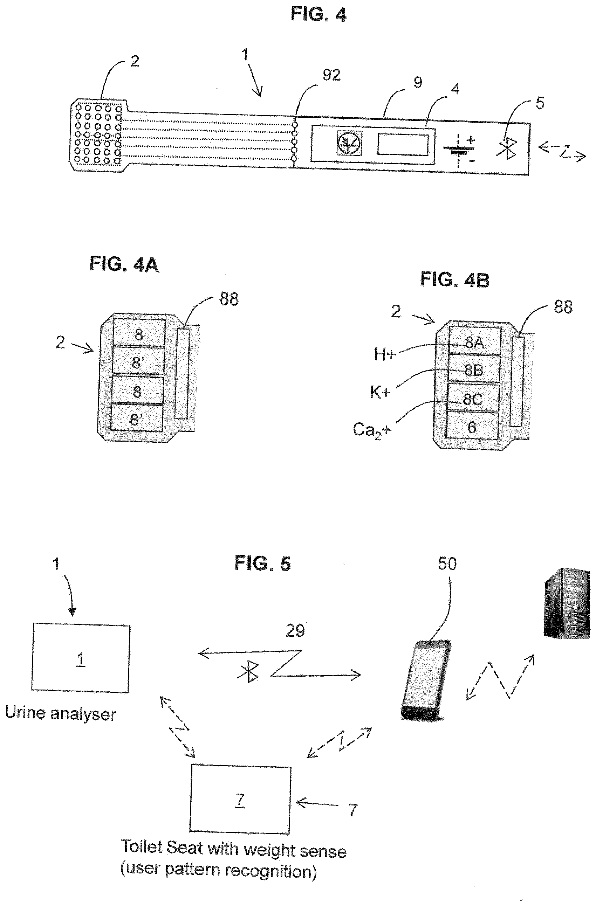

There may be provided more than one electro-chemical sensor, each specialized in detecting a subset of the above-mentioned substances, as illustrated at Fig 4A.

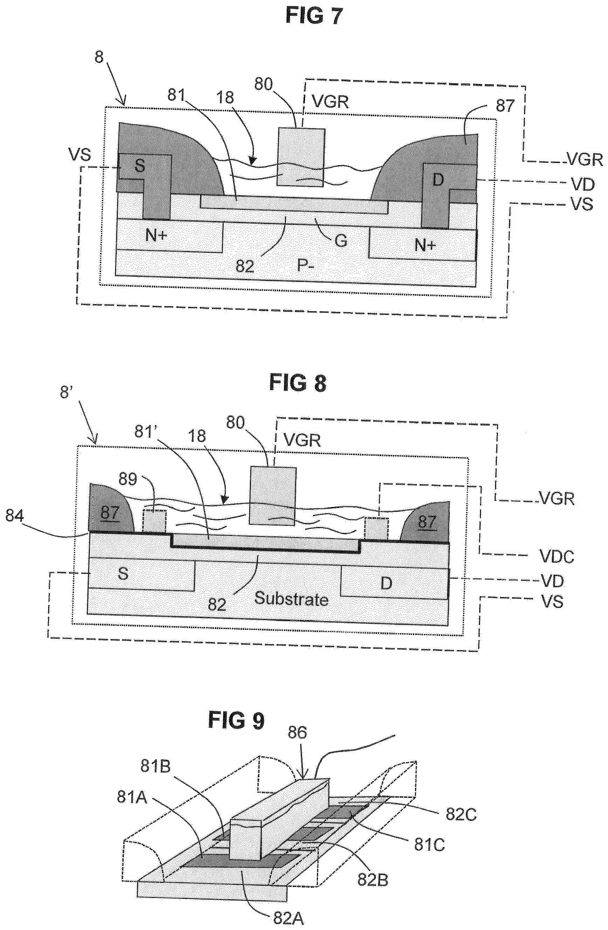

One particular type of electro-chemical sensor proposed is an **ion selective Field Effect Transistor** (abbreviated in 'ISFET'), denoted **8** and illustrated on figure 7. An ISFET may also be referred to as a chemical Field Effect Transistor (abbreviated as 'ChemFET').

ISFET principle relies on a silicon chip which, when placed in contact with a test solution, urine sample in the present case, detects and measures the variable voltage potential between its surface and underlying semiconductor material. This variable potential is proportional to the ion concentration in the sample, and is used to determine the concentration of the ions of interest.).

In the case of pH measurement, pH value is a function of H+ ion concentration. The ion sensitive membrane is formed by the semi-conductor itself (SiO2, Si3N4, Al203 or Ta2O5) and forms the gate port **82.**

In the case of other ions (Na+, Mg2+, Ca2+, K+, Cl-), a highly selective organic membrane **81** specific to the respective ion of interest is arranged on top of the gate semiconductor **82,** in other words the selective organic membrane **81** is interposed between the test solution (urine sample here) and the gate area.

Epoxy coating **87** isolates Drain and Source electrodes. As shown in Fig 7, such epoxy coating forms a cup in which the urine sample **18** is held opposite the gate area without interfering with the rest of the silicon layers, notably the substrate. Substrate is doped P, whereas source and drain pads are doped N in the disclosed non limitative configuration.

ISFET also requires a reference electrode **80,** which is connected to (coupled to) the gate area of the transistor. The reference electrode **80** may be held above the gate port of the transistor, so that a urine sample, when present, is interposed between the reference electrode and the ion sensitive membrane of the transistor.

In one embodiment, the reference electrode **80** contains a reference electrolyte solution placed in a small container having an exchange wall. In one embodiment, the container may be made of aluminum with the exchange wall being a ceramic foil. The reference electrode **80** may be placed on the same chip as the rest of the transistor or externally. The reference electrode **80** is electrically coupled to a gate reference voltage denoted **VGR.**

In some examples, the exchange of ions between the urine sample **18** and the electrolyte contained in the reference electrode creates a Gate trigger voltage at the gate port (G) of the transistor.

The reference electrode 80 may comprise a gel solution, or a gel composition. The solution contained therein is gradually consumed and/or gradually leaks out of the reference electrode.

According to one particular option, the leakage rate can be slowed if the reference electrode remains immersed in flushing water a certain time.

Alternatively, the reference electrode 80 may comprise a powder or a solid compound.

Alternatively, the reference electrode 80 may be constituted by a metallic compound, such as a metal wire.

In alternative example embodiments, the reference electrode **80** may be formed directly on the gate area of the transistor. For example, the reference electrode may be provided as metallic contact area formed on the gate area of the transistor. For example, the metallic contact area may be gold (Au) wafer. The gold wafer may be provided as a 1mm x 1mm square contact area. Other area sizes may be used as appropriate.

The concentration of ion of interest is reflected by the current flowing in the transistor, which is in turn measured by a voltage difference between the source and the gate of the transistor (VG-VS). This current and/or VG-VS voltage may be used by the electronic unit **4** to determine the ion concentration level.

### pH measurement

pH value is an index reflecting concentration of H+ ions. The ion sensitive membrane is the semiconductor made as a top deposited layer of the transistor assembly connecting the drain and the source.

The gate **G** portion of the transistor assembly is in direct contact with the test solution **18.** After each use, water from flushing is advantageously used to rinse the gate area of the ISFET. The flushing water is also advantageously used to recalibrate the sensor in case of drift with time, provided that the pH of tap water is known.

### Other ions

According to one option, there is provided an additional ISFET selective to other chemical species :
Normal range of (Mg2+) concentration is : [1.7 - 2.4] mg/day
   - Sodium (Na+)
Normal range of (Na+) concentration is : [100-260] mEq/day
   - Chloride (Cl-)
Normal range of (Cl-) concentration is : [80-250] mEq/day
Similarly specific membranes selective to let respectively Mg2+, Ca2+, K+,Na+,Cl- ions pass through are used.

### Calibration and recalibration

For each ISFET, the process of the periodic drop of calibration solution and subsequent recalibration can be used as explained before for H+ and flush water.

A calibration solution (tap water or a specific calibration solution) is brought into contact with the gate area and the measure is compared to an expected value, which allows to determine a deviation that is to be compensated for.

According to one option, the device may further comprise a calibration **solution reservoir 88** arranged above the **electrolyte sensor** 3 to periodically provide a drop of calibration solution on the ion selective Field Effect Transistor. The drop of such calibration solution is used to recalibrate the electrochemical sensor. This calibration solution may be tap water or a specific solution.

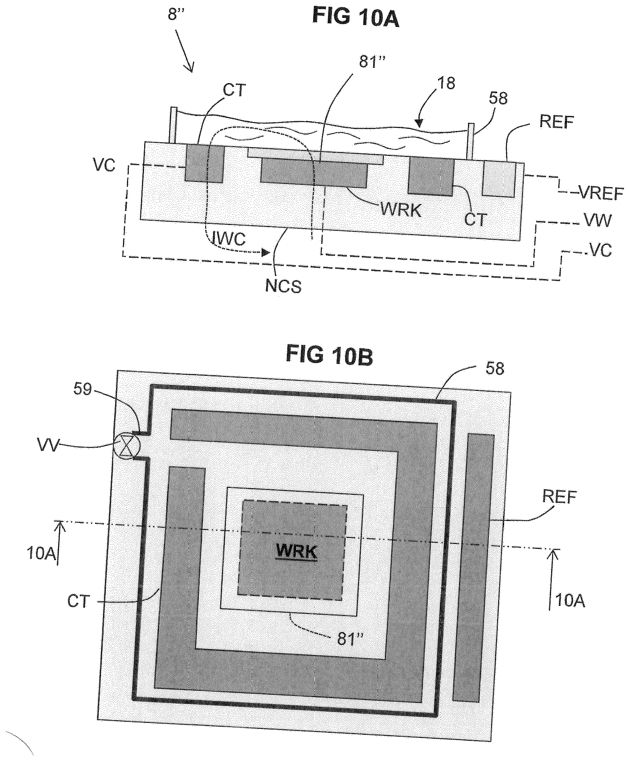

A miniaturized control valve (not shown at the figures except schematically at figure 10B) is used to release the above-mentioned drop of calibration solution. Such drop can be programmed to occur every month. Generally speaking, the recalibration with the drop preferably occurs every period comprised in the range [15 days - 45 days].

Another particular type of electro-chemical sensor proposed is a **biological selective Field Effect Transistor,** otherwise called enzyme selective Field Effect Transistor (abbreviated in 'enFET'), denoted **8'** and illustrated on figure 8.

The configuration is very similar what has been explained before regarding ISFET.

Filtering is here more elaborate and requires either an organic thin filtration membrane specifically configured to let some targeted molecules or a specific enzymatic membrane which reacts with the species of interest in the test sample. Reference **81'** denotes the enzymatic layer which let the chemical species of interest go through, or reacts with the species of interest in the test sample.

The reference electrode **80** is electrically coupled to a gate reference voltage denoted **VGR.** The exchange of ions between the urine sample **18** and the reference electrode electrolyte creates a Gate trigger voltage at the gate port (G) of the transistor. Alternatively, the reference electrode may be provided in any of the ways described above in relation to the ISFET electro-chemical sensor 8.

There may be provided optionally a surrounding counter electrode **89** which is set at a reference voltage VDC, which may be different from the reference voltage **VGR** of the reference electrode.

Epoxy coating **87** form a cup in which the urine sample **18** is held opposite the gate area without interfering with the rest of the silicon layers, notably the substrate. Here by contrast to the ISFET, the selective layer is more complicated to assemble; an intermediate foil denoted **84** is arranged on the silicon layers and the enzyme selective membrane 81' is arranged on top of the intermediate foil **84.**

### H₂O₂

The inventors have found that Urinary hydrogen peroxide (H₂O₂) is an amazingly good biomarker of oxidative stress, defined as the imbalance between Reactive Oxygen Species (ROS) production and antioxidant defense inside a human or animal organism. Oxidative stress is a risk factor playing a significant pathogenetic role for many diseases.

A specific membrane selective to let the H₂O₂ molecules pass through is used in a ISFET configuration.

Normal range of H₂O₂ in urine = 15 +/- 9,8 µmol/L

### Beta-HCG

Taking Beta-HCG Hormone (pregnancy marker) as another example, the enzymatic layer is designed to let only Beta-HCG Hormone pass across the membrane.

According to a particularly optimized configuration (not shown), there may be provided a reference electrode, a working electrode and a counter electrode, all arranged in a screen printed electrode configuration.

Generally speaking, there may be found several types FET sensor, either ISFET **8** or enFET **8'** in the reception area **2,** as illustrated in figure 4A, each one being selective to one ion type or one biological compound. Note that reference solution reservoir **88** may be either common to all the FET sensors, or there may be provided several individual reservoirs, or even a mix between the two solutions.

In a particular configuration, illustrated at figure 4B, there may be provided three ISFET devices, namely :
- one (ref 8A) for measuring the concentration of H+ ions to determine pH,
- one (ref 8B) for measuring the concentration of K+ ions to determine the Potassium level,
- one (ref 8C) for measuring the concentration of Ca2+ ions to determine Calcium level,

Of course one or more additional ISFETs for measuring the concentrations of other ion species can be added to this configuration. Alternatively, other combinations of two or more of ISFET 8' and enFET 8" electro-chemical sensors may be used.
According to a particularly optimized configuration shown at Figure 9, there may be provided 3 ion selective Field Effect Transistors, with a same reference electrode **86.** A first ion selective Field Effect Transistor has a selective membrane **81A** and an underlying gate **82A.** A second ion selective Field Effect Transistor has a selective membrane **81B** and a underlying gate **82B.** A third ion selective Field Effect Transistor has a selective membrane **81C** and a underlying gate **82C.**

Apart from the electrolyte sensor, there may be provided an optical sensor **6.** This optical sensor **6** is used to measure the opacity of the sampled urine, the color of the sampled urine and the concentration of the sampled urine. A light emitter directs light rays toward a urine sample contained in a cup **24**; a photodiode receives the transmitted light power (may include a reflection on a mirror) ; one or several color filters can be interposed. Optical analysis is known and therefore not detailed here. Urine density and color index can be assessed by the electronic unit 4; personal biological ratings corresponding to Urine density and color index can thus be followed by the user. Content of the cup 24 may be purged by means of a miniaturized electro-valve.

Regarding some mechanical aspects, the device can be monolithic as schematically shown at Figure 6A.

According to one variant shown at Fig 4, the device may be formed as two parts detachably associated to one another. A junction **92** with a connector joins the housing enclosing the electronic unit **4** and the reception area **2.** The reception part can be removed for deep cleaning or completely exchanged by a new one.

According to one option, there can be provided a degree of freedom between the holder member and the reception area, as illustrated at Fig 6B. Axis **A1** denotes a possible rotation motion to move the sensitive area closer to the center of the bowl. This movement can be powered and controlled by the electronic unit **4.**

However, at rest position, the urine reception area is preferably located adjacent to the internal wall of the bowl so it can receive at least partly some flushing water flow. There may be provided a deflector that helps directing the flushing flow toward the urine reception area in order to rinse the urine reception area. Additionally, there may be provided a purge valve that may be used to purge the content of the urine (or the water) retained inside the reception area; this also helps keeping the reference electrode **80** in a liquid to minimize leakage and extend its lifetime.

Alternatively the movement can be manually generated, with a handle to pull or a button to push and a linkage attached to a movable reception part.

The toilet in question may be used by several different users. Note that male and female hormones types and/or levels are always different; this allows to recognize if a male user or a female user is using the toilet.

The urine analyser device 1 can also monitor a number of miction(s) per specified time period, for example per night and/or per day.

One way to recognize one user among several users is to place a weight sensor in the toilet seat 7. Additionally or alternatively, there may be provided contact electrodes to perform an impedance measurement; A particular signature is allocated to each user of the toilet, after initial teach-in proceedings. When the weight is used, a rather narrow range is allocated to each user.

Another way is to provide a fingerprint sensing device 47.

Another way is to provide a microphone with a basic voice recognition function such that a user of the toilet can just say a word (first name, or "it's me", or "hello") which is enough to distinguish one particular user among a defined group of users (e.g. a family).

Another way is to provide contact ECG electrodes and a ECG analyzer in the toilet seat, with one electrode in contact with one thigh and another configured to be touched by a finger.

Whenever a urine sampling is performed with no user recognition, the results is qualified as 'non affected' in the corresponding smartphone application.

The system in which the urine analyzer device works includes a remote computing device **50.** Any generally speaking "portable electronic device" can also be used, such as a smartphone, atablet, a phablet, a PDA, a laptop computer, or any like wireless enabled device that can send and/or receive data through a wireless link **29.** An application corresponding to the urine analyzer device is provided in one or more smartphone(s) if several users are involved.

For each user, a timechart with number of miction per day/night, evolution of pH and concentration of main chemical substance(s) can be displayed at the smartphone application.

The optional microphone **19** can be used to sense a flush sound signature. This provides automaticity of cleaning and recalibrating after toilet flush.

Regarding practical use, a female user has to direct the flow of urine toward the reception area, helped by the above mentioned displacement of the sensitive area; a male user, whether sitting or standing, has to direct the flow of urine toward the reception area.

Another particular type of electro-chemical sensor proposed is a **3-electrode configuration,** denoted **8"** and illustrated on figures 10A,10B. There are provided 3 electrodes on a non conductive substrate denoted **NCS:**
- a reference electrode denoted **REF** (preferably kept at distance from the reaction site in order to maintain the substrate at a known and stable potential **VREF**),
- a working electrode denoted **WRK** through which a reactive current flow is sensed
- a counter electrode denoted **CT** which conducts the current flowing through the working electrode.

The working electrode serves as the transduction element in the biochemical reaction (also known as redox electrode); a selective or reactive membrane **81"** (similar as explained above for enFET) is interposed between the urine test sample and the working electrode.

A urine sample **18** is held momentarily in a containing volume delimited by a border **58.** At one location of the border there is provided a mouth **59** equipped with a controlled valve **VV**.

The current **IWC** flowing between the respective terminals **VW,VC** of working electrode and the counter electrode reflects the concentration of the species of interest.

The reference electrode is typically made from Ag/AgCl.

The working electrode is made of gold, carbon, diamond, platinum or any other metal not prone to corrosion.

According to one option, the working electrode is preferably a boron doped diamond electrode.

Although the example in Figure 9 showed three ion selective Field Effect Transistors, a common reference electrode 86 may be similarly used for any combination of two or more or any of ISFET 8, enFET 8', and 3-electrode configuration 8" electro-chemical sensors 3. The electronic unit 4 may further comprise a multiplexer (not shown in the figures) so that the individual outputs for each sensor can be read from the common reference electrode 86.

As described above, the urine analyser device 1 may comprise any combination of two or more of the different types of electro-chemical sensors 3 i.e. ISFET 8, enFET 8' or 3-electrode configuration 8". The urine analyser device 1 may have a modular construction so that a number of electro-chemical sensors may be removably couplable to the device. For example, a user can manually insert or otherwise couple and remove the combinations of electro-chemical sensors based on which substance(s) of interest the user wants to monitor. The device may be configured to accommodate two, three, four, five or more electro-chemical sensors. It is to be appreciated that a user may select to use fewer electro-chemical sensors than the device can accommodate. For example a device configured to accommodate three electro-chemical sensors, may comprise one, two or three electro-chemical sensors. At times, the device may house no electro-chemical sensors at all; for example, a user may temporarily remove all of the electro-chemical sensors for replacement, transportation or thorough cleaning. The urine analyser device 1 may be configured to detect when an electro-chemical sensor is newly coupled to the device which may trigger the calibration operation as described above.

Information about known disorders and dysfunctions associated with concentration levels of various substances found in urine can be stored in a memory of the remote computing device 50 and/or a remote database that the computing device 50 may be configured to communicate with. The remote computing device 50 and the database may respectively be implemented as one or more of a single computing device, a cloud server network of computing devices, and a distributed peer-to-peer network of computing devices. Some examples of known disorders and dysfunctions were given in the background section. It is to be appreciated that the listed disorders and dysfunctions are examples only. In examples, the information stored at the remote computing device 50 and/or the database may be updated about which substances and/or concentrations of substances, taken alone or in combination, could indicate a particular disorder or dysfunction of a human or animal body.

By comparing the data it receives from the urine analyzer device with the information it has access to, the remote computing device 50 may be configured to determine a disorder and/or dysfunction the user may have. Thus the remote computing device 50 may be configured to communicate user feedback to one or more of the urine analyser and other remote user devices such as a smartphone, tablet, smart wearable device, personal computer, laptop computer, smart television, etc. Because the information at the remote computing device 50 and/or database can be updated over time, the overall system remains relevant. For example information about newly discovered associations between concentrations of one or more substances and particular disorders and/or dysfunctions can be added to the remote computing device 50 and/or the database. For example, in the future medical associations, regulators or the like may determine that a certain concentration of Na+ is likely associated with a particular type of cancer. Therefore information defining this association can provided as an update to the remote computing device 50 and/or the database.

For example, a high sodium (or salt) consumption (determined from a concentration of Na+) and a low concentration of K+ may be associated with an eventual development of cardiovascular diseases. Cross referencing the measured concentrations of the different substances (in this example, the respective concentrations of Na+ and K+ ions) can allow for a much more accurate determination of potential disorders and/or dysfunctions. A high concentration of Na+ taken alone can be associated with hypertension, whereas a high concentration of Na+ and a low concentration of K+ can provide a stronger indicator of increased risk of cardiovascular diseases such as strokes.

If there is an incoherence between different measured values, e.g. a very low Na+ concentration level and a very low K+ concentration level, this could be an indication of an error. In the case of Na+ and K+, one would typically expect to find an inversely proportional relationship between the two values. The urine analyser device 1 may be configured to evaluate the record and delete the particular reading and/or report it as false. Similarly, the user may manually choose to delete the record and/or report a reading as false. In response, the remote computing device 50 may perform a further evaluation to confirm that the reading is in fact an anomaly or may be configured to communicate an alert to one or more of the urine analyser device 1 and/or other remote user devices if the incoherence persists for future measurements.

If information about existing associations between concentrations of one or more substances and particular disorders and/or dysfunctions are subsequently seen to be incorrect or inaccurate, then that information can be removed from the remote computing device and/or database. Given the importance of this addition or removal of information, secure administrator access rights can be setup so that only authorised personnel may access the remote computing device 50 and/or the database.

It should be appreciated that there may be a margin of error in the sensor data. Therefore feedback from the remote computing device 50 may comprise a message altering the user to consult a medical professional e.g. their doctor, a local hospital or clinic. Additionally or alternatively, the remote computing device 50 may be configured to communicate directly with a user's registered local doctors' surgery, hospital and/or clinic. For example, the feedback may be communicated to and stored in a record associated with the user's local surgery system (e.g. a database, server or distributed cloud servers). A medical professional can then access the user's records using a device configured to securely connect to the record system. If the remote computing device 50 determines that the disorder and/or dysfunction should be flagged as "serious" and requiring immediate attention, then one or more push notification alerts may be pushed to the medical professional's device, e.g. by the record system generating an appropriate alert to be pushed to a connected device.

The time it takes for an ISFET electro-chemical sensor 8 or a 3-electrode electro-chemical sensor 8" to react to the test solution 18 (e.g. urine sample) or the calibration solution, may range from several seconds in the absence of a selective membrane (e.g. less than 60 seconds) to several minutes in the presence of a selective membrane. An enFET electro-chemical sensor 8' may react more slowly, taking longer for the electronic unit 4 to determine a concentration measurement of the substance of interest. For the ISFET 8 or enFET 8' configurations, the reaction time T is defined as the period of time it takes from when the sample solution 18, or calibration solution, comes into contact with the electro-chemical sensor, to when the current flow and/or VG-VS voltage reflects the concentration of the substance of interest. For the 3-electrode configuration 8", the reaction time is defined as the period of time it takes from when the sample solution 18, or calibration solution, comes into contact with the electro-chemical sensor, to when the current flow IWC between the respective terminals VW, VC of the working electrode and the counter electrode reflects the concentration of the substance of interest.

As described above, the electro-chemical sensor(s) 8, 8', 8" may be hydrated, for example by rinsing with toilet flush water after the device is used. Keeping an electro-chemical sensor hydrated will help to reduce its reaction time T, so that the electronic unit 4 can determine the concentration of the substance of interest faster than without hydration. By keeping the reference electrode 80, 86 hydrated, the reaction time may be reduced to an order of seconds, for example less than 60 seconds, compared to several minutes, for example approximately ten minutes. In examples in which the toilet flush water is chlorinated, i.e. comprises Cl-, this will also help to preserve the stability of the reference electrode 80, 86. It is to be appreciated there may be some variation in the reaction times based one or more of: what substance the electro-chemical sensor is configured to detect; the level of hydration of the electro-chemical sensor; and one or more characteristics of the toilet flush water, which may or may not comprise one or more further liquids.

The toilet flush water may also help to clear or clean residual depositions from electro-chemical sensors 8, 8', 8", which may help to regenerate the occupied sensor sites. This may help to maintain the sensitivity of the electro-chemical sensors 8, 8', 8" and thus help maintain the accuracy of the measurements they make.

In some of the above examples and as shown in Fig 4A, there may be provided more than one electro-chemical sensor, each respectively configured to measure a concentration of a particular substance. The following examples are directed to a urine analyser device 1 as previously described, but in this instance comprising at least two ion selective Field Effect Transistors (ISFET) 8, wherein a first ISFET 8 is configured to measure a concentration of Na+, and a second ISFET 8 is configured to measure a concentration of K+. Each ISFET 8 may respectively comprise any of the ISFET features as described above. However it is to be appreciated that the device is not limited to these two specific types of electro-chemical sensors, and may comprise further additional electro-chemical sensors of any of the types described herein, and/or any may further comprise other features of the urine analyser device 1 as described herein.

Improved accuracy in analysis of K+ or Na+ concentration levels, as measured by the respective first or second ISFET 8, may be achievable if a number of measurements are obtained for a user over a period of time. For example, the period of time may be 24-hours, although other time periods may be used. Furthermore, each concentration measurement may be adjusted (e.g. normalized) to remove the impact of water dilution. Water dilution is a source of error when the remote computing device 50 analyses the measurement data it receives e.g. the K+ and Na+ concentration measurements. The adjustment (normalization) may be performed by the remote computing device 50 by using a measurement of concentration of Creatinine for each respective urine sample. For example, the Creatinine concentration of a urine sample for may be measured by an enFET 8' electro-chemical sensor, and determined by the electronic unit 4. The Creatinine concentration measurement may be transmitted by the coupler 5 of the analyser device 1 to the remote computing device 50 along with the respective K+ and Na+ concentration measurements.

The enFET 8' electro-chemical sensor configured to measure the concentration of Creatinine may be an integral or modular part of the urine analyser device 1, alternatively it may be a separate, dedicated device. A separate, dedicated device may be configured to communicate its measurement data to the remote computing device 50, for example directly or via another device such as the urine analyser device 1.

It is known that Creatinine is excreted in urine, usually with a substantially constant quantity per individual or population group. Therefore the quantity excreted goes through the same dilution factor as the substance(s) of interest i.e. in this example, the Na+ and K+ ions measured by the first and second ISFET 8, respectively. Therefore, by normalizing the measurement of the substance by Creatinine, the remote computing device 50 can be configured to extrapolate an estimate for the quantity of the substance(s) (e.g. ions) from the total concentration.

Alternatively or in addition to using an electro-chemical sensor to measure the concentration of Creatinine in urine, an optical sensor 6 as described may be used to estimate the concentration of Creatinine. The optical sensor may also be configured to measure one or more optical properties of the urine sample. For example the optical properties may include any one or more of: the colour of the urine sample, a level of absorbance of light by the urine sample, and a level of transmittance of light by the urine sample, and the opacity of the urine sample.

The optical sensor 6 may be an integral or modular part of the urine analyser device 1. The optical property measurement data may be transmitted by the urine analyser device 1 to the remote computing device 50 along with the respective K+ and Na+ concentration measurements. Alternatively, the optical sensor 6 may be a separate, dedicated device that is configured to work in co-operation with the urine analyser device. A separate, dedicated optical sensor 6 device may be configured to communicate the optical property measurement data to the remote computing device 50 either directly or via the urine analyser device 1.

The remote computing device 50 may be configured to compare the received optical property measurement data with predetermined optical property data. The predetermined optical property data may be stored at the remote computing device 50 and/or at a remote database (as described above) that the remote computing device 50 is configured to communicate with. The predetermined optical property data may also define associations between the predetermined optical property data and general concentration levels of urine. For example, certain predetermined optical properties may be associated with a respective corresponding concentration of urine, for example based on a colour index. Such a colour index may be stored in a memory of the remote computing device 50 and/or the remote database. Thee colour index may also be regularly updated and personalised for a user by the optical sensor 6 device transmitting colour data (and/or other optical properties of the urine) to the remote computing device and/or database, as part of the recurring calibration operation.

The general concentration levels of urine may in turn may be associated with a respective corresponding predetermined Creatinine concentration level. Predetermined Creatinine concentration levels may be stored at the remote computing device 50 and/or at a remote database in the same way as the predetermined optical property data.

The remote computing device 50 may be configured to compare received optical property measurement data with the predetermined optical property data and based on said comparison, determine a general concentration level of the urine sample. In turn, and based on the determined general concentration level, the remote computing device 50 may be configured determine a corresponding Creatinine concentration level. Alternatively, the remote computing device 50 may be configured to compare the received optical property measurement data directly with the predetermined Creatinine concentration level associations, bypassing the need for a determining a general concentration level of the urine sample.

In an alternative example, the estimation of the Creatinine concentration level may be determined locally by the electronic unit 4 of the urine analyser device 1. That is, the electronic unit 4 may be configured to associate optical properties as measured by the optical sensor 6 to predetermined concentration levels of Creatinine. The electronic unit 4 may be programmed with predetermined data (e.g. colour-index data) to enable it make the associations. Thus an estimate of a concentration level of Creatinine in a urine sample can be determined locally and transmitted to the remote computing device 50 and/or database, as described above.

The remote computing device 50 may therefore be configured to use the determined estimate of the Creatinine concentration level of a urine sample to adjust (normalize) the received ion concentration measurement of the K+ or Na+ from the first or second ISFET 8.

In an alternative example, the remote computing device 50 may be configured to interpret received ion concentration measurement data without the need to adjust (normalize) the data based on a determined (or estimated) Creatinine concentration level. For example, for a current urine sample, the remote computing device 50 may be configured to calculate a ratio of the received ion concentration of Na+ to the received ion concentration of K+ (Na+ : K+). There is a synergistic effect between the concentration level of sodium ion (Na+) and the concentration level of potassium ion (K+) in the urine. Potassium levels often change with sodium levels and there is an inverse correlation between the two due to internal mechanisms of the human (or animal) body. There is also a positive correlation between the ratio of the concentration of Na+ to K+, and the risk of hypertension. For example a ratio value of greater than 1 implies that the user has a high intake in salt relative to potassium, which is not recommended because high levels of salt in the human (or animal) body have been shown to be contributing factor to cardiovascular diseases.

The remote computing device 50 may be configured to associate the result of the ratio with one or more lifestyle recommendations, warnings or prompts. For example if it is determined that the ratio is above a threshold level indicating that the user is taking in too much sodium (or salt), then the remote computing device 50 can generate and send an appropriate feedback to the urine analyser device 1 and/or further user device e.g. "sodium (or salt) intake is high: try reducing your sodium (or salt) intake to X grams per day; drink more water; eat/drink a high-in-potassium food or drink e.g. eat a banana". Conversely, it is to be appreciated if the sodium (or salt) intake is determined to be too low, e.g. below a threshold value (or if the potassium intake is too high), then the remote computing device 50 can generate and send an appropriate feedback to the urine analyser device 1 and/or further user device e.g. "sodium (or salt) intake is low: eat/drink a high-in-sodium food or drink e.g. eat/drink some celery, beets and/or milk".

Alternatively or in addition, the control unit 4 may be configured to calculate locally the ratio of the concentration of Na+ to K+. Further, the control unit 4 may be programmed to generate the user feedback for output by the urine analyser device 1 e.g. via visual, audio or haptic feedback means, and/or for transmission to one or more of the remote user devices.

The urine analyser device 1 described in the preceding examples may comprise a controller 40, for example as part of the electronic unit 4, as illustrated in Figs. 3 and 4. Implementation of a controller 40 may be as controller circuit. The controller 40 may be implemented in hardware alone, have certain aspects in software including firmware alone or can be a combination of hardware and software (including firmware).

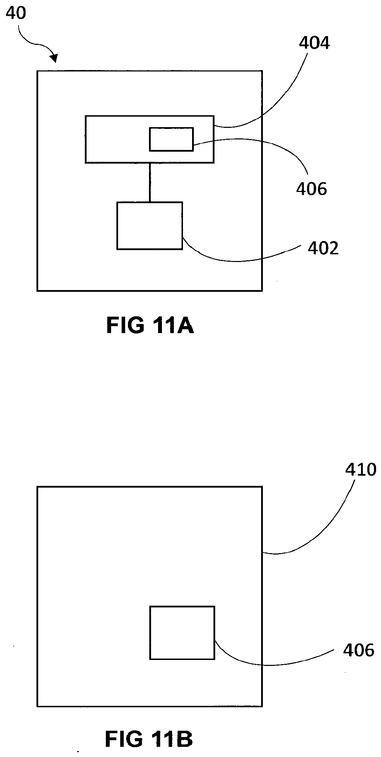

As illustrated in Fig 11a the controller 40 may be implemented using instructions that enable hardware functionality, for example, by using executable instructions of a computer program 406 in a general-purpose or special-purpose processor 402 that may be stored on a computer readable storage medium (disk, memory etc) to be executed by such a processor 402.

The processor 402 is configured to read from and write to the memory 404. The processor 402 may also comprise an output interface via which data and/or commands are output by the processor 402 and an input interface via which data and/or commands are input to the processor 402.

The memory 404 stores a computer program 406 comprising computer program instructions (computer program code) that controls the operation of the urine analyser device 1 when loaded into the processor 402. The computer program instructions, of the computer program 406, provide the logic and routines that enables the urine analyser device 1 to perform techniques as described herein. The processor 402 by reading the memory 404 is able to load and execute the computer program 406.

As illustrated in Fig 11b, the computer program 406 may arrive at the urine analyser device 1 via any suitable delivery mechanism 410. The delivery mechanism 410 may be, for example, a non-transitory computer-readable storage medium, a computer program product, a memory device, a record medium such as a compact disc read-only memory (CD-ROM) or digital versatile disc (DVD), an article of manufacture that tangibly embodies the computer program 406. The delivery mechanism may be a signal configured to reliably transfer the computer program 406. The urine analyser device 1 may propagate or transmit the computer program 406 as a computer data signal.

Although the memory 404 is illustrated as a single component/circuitry it may be implemented as one or more separate components/circuitry some or all of which may be integrated/removable and/or may provide permanent/semi-permanent/ dynamic/cached storage.

Although the processor 402 is illustrated as a single component/circuitry it may be implemented as one or more separate components/circuitry some or all of which may be integrated/removable. The processor 402 may be a single core or multi-core processor.

A computer readable medium 410 therefore comprises computer program code 406 stored thereon, the computer readable medium and computer program code 406 being configured to, when run on at least one processor 402, perform:
causing at least one electro-chemical sensor (3) to measure a quantity of at least one substance contained in urine, such as a physiological compound or a chemical component;
causing a coupler (5) to send resulting measured data from the at least one electro-chemical sensor to a remote computing device 50;
wherein the wherein the electro-chemical sensor (3) comprises at least one of:
   an ion selective Field Effect Transistor (ISFET) (8) for sensing levels of one or more physiological ions;
   an enzyme selective Field Effect Transistor (enFET) (8') for sensing levels of one or more physiological compounds or chemical components; and
   a 3-electrode sensing unit for sensing levels of one or more physiological compounds or chemical components.

The urine analyser device 1 therefore comprises:
at least one processor 402; and
at least one memory 404 including computer program code
the at least one memory 404 and the computer program code configured to, with the at least one processor 402, cause the urine analyser device 1 at least to perform:
   causing at least one electro-chemical sensor (3) to measure a quantity of at least one substance contained in urine, such as a physiological compound or a chemical component;
   causing a coupler (5) to send resulting measured data from the at least one electro-chemical sensor to a remote computing device 50;
   wherein the wherein the electro-chemical sensor (3) comprises at least one of:
      an ion selective Field Effect Transistor (ISFET) (8) for sensing levels of one or more physiological ions;
      an enzyme selective Field Effect Transistor (enFET) (8') for sensing levels of one or more physiological compounds or chemical components; and
      a 3-electrode sensing unit for sensing levels of one or more physiological compounds or chemical components.

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

Where a structural feature has been described, it may be replaced by means for performing one or more of the functions of the structural feature whether that function or those functions are explicitly or implicitly described.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one..." or by using "consisting".

In this brief description, reference has been made to various examples. The description of features or functions in relation to an example indicates that those features or functions are present in that example. The use of the term 'example' or 'for example' or 'may' in the text denotes, whether explicitly stated or not, that such features or functions are present in at least the described example, whether described as an example or not, and that they can be, but are not necessarily, present in some of or all other examples. Thus 'example', 'for example' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example but does not necessarily have to be used in that other example.

Although embodiments of the present invention have been described in the preceding paragraphs with reference to various examples, it should be appreciated that modifications to the examples given can be made without departing from the scope of the invention as claimed.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavouring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

Further embodiments of the invention are defined in the following clauses.
{1} A urine analyzer device (1) configured to be installed in a toilet bowl (10), the device comprising:
   - a holder member (9), to attach the device to the toilet bowl,
   - an electronic unit (4), enclosed in a housing,
   - a urine reception area (2), with at least one electro-chemical sensor (3), configured to measure a quantity of at least one substance contained in urine, such as a physiological compound or a chemical component,
   - a coupler (5) configured to send resulting measured data from the at least one electro-chemical sensor to a remote computing device (50),
      wherein the electro-chemical sensor (3) comprises at least one of:
      an ion selective Field Effect Transistor (ISFET) (8) configured to sense levels of one or more physiological ions;
      an enzyme selective Field Effect Transistor (enFET) (8') configured to sense levels of one or more physiological compounds or chemical components; and
      a 3-electrode sensing unit (8") configured to sense levels of one or more physiological compounds or chemical components.
{2} The device of clause {1} wherein the ion selective Field Effect Transistor comprises a reference electrode (80) coupled to a gate port (G) of the transistor, the ion selective Field Effect Transistor (8) configured so that when a urine sample is present, the urine sample is interposed between the reference electrode and the gate port (G) of the transistor.
{3} The device of clause {2} wherein the ion selective Field Effect Transistor is selective to hydrogen ions (H+), whereby the ion selective Field Effect Transistor (8) comprises an ion sensitive membrane for measuring the pH of a urine sample, so that in the presence of a urine sample, the ion sensitive membrane is interposed between the urine sample and the gate port (G) of the transistor.
{4} The device of clause {3}, further comprising at least two additional ion selective Field Effect Transistors:
   - one for measuring the concentration of Ca2+ ions to determine Calcium level in urine,
   - one for measuring the concentration of K+ ions to determine the Potassium level in urine, each of the additional ion selective Field Effect Transistorscomprising a respective ion selective membrane interposed between a urine sample, when present, and the gate port (G) of the transistor.
{5} The device of any of clauses {2} to {4}, comprising at least two ion selective Field Effect Transistors (8), wherein:
   - a first ion selective Field Effect Transistor (8) is configured to measure a concentration of Na+ ions to determine Sodium level in urine,
   - a second ion selective Field Effect Transistor (8) is configured to measure a concentration of K+ ions to determine the Potassium level in urine, and
      wherein the first and second ion selective Field Effect Transistors (8) comprise an ion selective membrane for measuring Na+ and K+ respectively, so that in the presence of a urine sample, the ion selective membrane is interposed between the urine sample and the gate port (G) of the respective transistor.
{6} The device of clause {5}, wherein the electronic unit (4) is configured to:
   for a urine sample, calculate a ratio of the concentration of Na+ ions to the concentration of K+ ions (Na+ : K+); and
   based on the calculated ratio, generate a user feedback for output by one or more of the urine analyser device and a remote user device.
{7} The device of any preceding clause, wherein the electro-chemical sensor (3) is configured to be hydrated at least by water from the flush of the toilet.
{8}. The device of any preceding clause further comprising a calibration solution reservoir (88) arranged above the electro-chemical sensor (3) to periodically provide a drop of calibration solution on the gate area of the ion selective Field Effect Transistor.
{9}. The device of clause {1}, comprising two or more electro-chemical sensors, wherein at least two of the electro-chemical sensors are connected to a same reference electrode (80).
{10}. The device of clause {1} wherein the 3-electrode sensing unit (8"), comprises a reference electrode, a working electrode and a counter electrode, arranged in a screen printed electrode configuration.
{11} The device of any preceding clause wherein the urine reception area (2) comprises a porous structure that can be wetted by a flow of urine excreted by a user, and rinsed subsequently by the flush of the toilet.
{12} The device of any preceding clause wherein the electronic unit (4) is at least partly housed in the holder member (9).
{13} The device of any preceding clause wherein the urine reception area (2) is movable with respect to the holder member 9 such that the urine reception area can be located closer to the center of the bowl upon actuation from the user.
{14} The device of any preceding clause, comprising at least one enzyme selective Field Effect Transistor (enFET) (8') wherein:
   at least one of the enzyme selective Field Effect Transistor (8') is configured to measure a concentration level of Creatinine in a urine sample.
{15} The device of clause {14}, wherein the coupler (5) is configured to send the measured Creatinine concentration level to enable the remote computing device (50) to normalize the measured data from the at least one electro-chemical sensor (3) for the same respective urine sample.
{16} The device of any preceding clause, further comprising an optical sensor (6), configured to measure one or more optical properties of a urine sample comprising: the colour of the urine, a level of absorbance of light by the urine, a level of transmittance of light by the urine, and the opacity of the urine;
   wherein the electronic unit (4) is configured to analyse the measured one or more optical properties of the urine sample and, based on the analysis, associate the urine sample with one of a plurality of predefined concertation levels of Creatinine, thereby generating an estimated Creatinine concertation level for the urine sample.
{17} The device of clause {16}, wherein the coupler (5) is configured to send the estimated Creatinine concentration level for the urine sample to enable the remote computing device (50) to normalize the measured data from the at least one electro-chemical sensor (3) for the same respective urine sample.
{18} The device of any preceding clause, wherein the urine reception area (2) is detachably mounted to the holder member.
{19} The device of any preceding clause, further comprising a microphone (19) and/or a basic fingerprint sensor (47) arranged on the portion of the device located outside the toilet bowl.
{20} A system comprising a device according to clause {1} and a toilet seat (7) comprising weight sensing elements and impedance measurement elements, configured to recognise a particular user pattern among a plurality of user patterns, and to allocate results of urine analysis to the recognised particular user.
{21} A method comprising:
   causing at least one electro-chemical sensor to measure a quantity of at least one substance contained in urine, such as a physiological compound or a chemical component;
   causing a coupler to send resulting measured data from the at least one electro-chemical sensor to a remote computing device;
   wherein the wherein the electro-chemical sensor comprises at least one of:
      an ion selective Field Effect Transistor (ISFET) for sensing levels of one or more physiological ions;
      an enzyme selective Field Effect Transistor (enFET) for sensing levels of one or more physiological compounds or chemical components; and
      a 3-electrode sensing unit for sensing levels of one or more physiological compounds or chemical components.
{22} A computer readable medium comprising computer program code stored thereon, the computer readable medium and computer program code being configured to, when run on at least one processor, perform:
   causing at least one electro-chemical sensor to measure a quantity of at least one substance contained in urine, such as a physiological compound or a chemical component;
   causing a coupler to send resulting measured data from the at least one electro-chemical sensor to a remote computing device;
   wherein the wherein the electro-chemical sensor comprises at least one of:
      an ion selective Field Effect Transistor (ISFET) for sensing levels of one or more physiological ions;
      an enzyme selective Field Effect Transistor (enFET) for sensing levels of one or more physiological compounds or chemical components; and
      a 3-electrode sensing unit for sensing levels of one or more physiological compounds or chemical components.

## Claims

1. A urine analyzer device (1) configured to be installed in a toilet bowl (10), the device comprising:
- a holder member (9), to attach the device to the toilet bowl,
- an electronic unit (4), enclosed in a housing,
- an optical sensor (6) to measure one or more optical properties of urine,
- a coupler (5) configured to send measured data to a remote computing device (50).

2. The device of claim 1, further comprising a urine reception area (2), where the optical sensor is to measure one or more properties of the sampled urine of the urine reception area (2).

3. The device of claim 2, wherein the urine reception area (2) forms a cup and the optical sensor (6) comprises a light emitter, which is configured to emits lights rays toward a urine sample contained in a cup (24, and a photodiode.

4. The device of any of claims 1-3, wherein the optical sensor is integral of the urine analyzer device.

5. The device of any of claim 1-4, wherein the optical sensor is a modular part of the urine analyzer device (1).

6. The device of any of claim 1-5, wherein one or more optical properties are: colour of the urine, level of absorbance of light by the urine, level of transmittance of light by the urine, opacity of the urine.

7. The device of any of claims 1-6, wherein the electronic unit is configured to associate optical properties as measured by the optical sensor (6) to predetermined concentration levels of creatinine.

8. The device of any of claims 1-7, wherein the electronic unit is configured to analyze the measured one or more optical properties of the urine sample.

9. The device of any of claim 1-8 claim 1, wherein the housing is watertight.

10. The device of any of claims 1-9, further comprising a urine reception area (2), with at least one electro-chemical sensor (3), configured to measure a quantity of at least one substance contained in urine, such as a physiological compound or a chemical component, wherein the electro-chemical sensor (3) comprises at least one of:
an ion selective Field Effect Transistor (ISFET) (8) configured to sense levels of one or more physiological ions;
an enzyme selective Field Effect Transistor (enFET) (8') configured to sense levels of one or more physiological compounds or chemical components; and
a 3-electrode sensing unit (8") configured to sense levels of one or more physiological compounds or chemical components.

11. A system comprising a device according to any of claims 1-10 and a remote computing device (50), wherein the urine analyzer device sends the optical property measurement data to the remote computing device (50).

12. The system of claim 11, wherein the remote computing device (50) is configured to compare data received from the device with predetermined optical property data stored at the remote computing device (50) and/or at a remote database with which the remote computing device is configured to communicate with.

13. The system of claim 12, wherein the remote computing device (50) is configured to determine a general concentration level of the urine.
